# FASCICULE DE BREVET EUROPEEN

(11) **EP 4 408 591 B1**
(45) Date de publication et mention de la délivrance du brevet: **12.11.2025**
(21) Numéro de dépôt: 22792856.1
(22) Date de dépôt: 26.09.2022
(51) Int. Cl.: B05B 11/00, A61M 11/00, A61M 15/08, B05B 15/14, B05B 11/02

(54) **ENSEMBLE DE DISTRIBUTION DE PRODUIT FLUIDE**
ANORDNUNG ZUR ABGABE EINES FLÜSSIGEN PRODUKTS
ASSEMBLY FOR DISPENSING A FLUID PRODUCT

(30) Priorité: 27.09.2021 FR 2110160
(43) Date de publication de la demande: 07.08.2024
(73) Titulaire: APTAR France SAS, 27110 Le Neubourg (FR)
(72) Inventeur: DESCHAMPS, Olivier, 27610 Romilly sur Andelle (FR); ESPINOZA, Diego, 76350 Oissel (FR); BLONDEL, Mathilde, 27400 Louviers (FR); REBLA, Naoufal, 27400 Louviers (FR)
(74) Mandataire: CAPRI
(86) Numéro de dépôt international: PCT/FR2022/051798
(87) Numéro de publication internationale: WO 2023/047067

(56) Documents cités:
- EP-A1- 2 939 700
- WO-A1-00/71263
- US-A- 5 547 131
- US-A1- 2005 098 172
- US-B1- 11 065 392

## Description

La présente invention concerne un ensemble de distribution de produit fluide.

Les dispositifs de distribution de produit fluide sont bien connus. Ils comportent généralement un réservoir, des moyens de distribution et une tête de distribution. La tête de distribution peut être une tête de pulvérisation, par exemple du type nasale. Ces têtes de pulvérisation comportent généralement deux pièces, à savoir un corps formant la tête et une pièce formant le gicleur, c'est-à-dire la partie qui crée la pulvérisation en faisant tourbillonner le produit fluide lorsqu'il est distribué. Dans le cas d'un gicleur externe, un petit manchon vient s'emmancher par l'extérieur dans l'extrémité aval du corps pour définir avec celui-ci ledit profil de pulvérisation. Dans le cas d'un gicleur dit interne, c'est un insert inséré par l'intérieur de la tête qui vient coopérer avec la paroi d'extrémité du corps pour définir le profil de pulvérisation. Dans les deux cas, les profils de pulvérisation comprennent généralement une pluralité de canaux non radiaux, notamment trois, formés soit dans la paroi de fond de la tête, soit sur le manchon formant gicleur externe, soit sur l'insert formant gicleur interne. Généralement, les têtes de pulvérisation sont assemblées sur des tiges de pistons de pompes ou des soupapes de valves pour guider le produit, qui est distribué hors de la pompe ou de la valve, vers l'orifice de pulvérisation. Toutefois, il a aussi été proposé de disposer une tête de pulvérisation directement sur la sortie d'un réservoir de type seringue pour pulvériser le contenu de ladite seringue plutôt que de l'injecter à travers une aiguille. Le document WO0071263 décrit un tel arrangement. A nouveau, ces têtes de pulvérisation sont toujours réalisées avec deux ou plusieurs pièces assemblées les unes avec les autres. Cette mise en œuvre empêche notamment l'utilisation de ces seringues en mode aspiration. En particulier, une seringue avec une tête de pulvérisation ne permet pas le mode aspiration typique des seringues, par exemple pour aspirer un produit fluide à partir d'un réservoir de prélèvement. Par ailleurs, une seringue avec une aiguille ne permet pas une pulvérisation du produit fluide lors de sa distribution.

Le document WO2015104511 décrit un ensemble de distribution de produit fluide comportant une pièce monobloc formant réservoir et aiguille de prélèvement, destiné à reconstituer un produit fluide en mélangeant d'abord une poudre avec un solvant, puis en pulvérisant ledit mélange sous forme d'un spray nasal, en une ou deux doses, à travers une tête de pulvérisation assemblée autour de l'aiguille de prélèvement. Cet ensemble présente des inconvénients. Ainsi, la pointe de l'aiguille de prélèvement n'est soit pas protégée, lorsque la tête de pulvérisation n'est pas assemblée, soit sous contrainte dans ladite tête de pulvérisation assemblée. Dans les deux cas, il y a un risque d'endommagement lors du transport de l'ensemble, avant utilisation, notamment en cas de chute de l'ensemble. De plus, la réalisation monobloc du réservoir avec l'aiguille peut être compliquée, et requiert d'utiliser le même matériau, ce qui n'est pas toujours souhaitable.

Les documents US5547131, US11065392, US2005098172 et EP2939700 décrivent d'autres dispositifs de l'état de la technique.

La présente invention a pour but de fournir un ensemble de distribution de produit fluide qui ne reproduit pas les inconvénients susmentionnés.

La présente invention a notamment pour but de fournir un ensemble de distribution de produit fluide qui garantit l'intégrité de la pointe de l'aiguille avant utilisation, notamment lors du transport et du stockage.

La présente invention a également pour but de fournir un tel ensemble de distribution de produit fluide qui permet d'optimiser les matériaux, notamment pour réaliser le réservoir et l'aiguille de prélèvement.

La présente invention a aussi pour but de fournir un ensemble de distribution de produit fluide qui soit simple et peu coûteux à fabriquer et à assembler, et qui puisse fonctionner à la fois en mode aspiration et en mode expulsion.

La présente invention a également pour but de fournir un tel ensemble de distribution de produit fluide qui permet de réaliser une pulvérisation correcte et reproductible.

La présente invention a ainsi pour objet un ensemble de distribution de produit fluide comportant un réservoir destiné à contenir un produit fluide et un piston coulissant dans ledit réservoir, une aiguille de prélèvement étant fixée sur ledit réservoir pour aspirer un produit fluide à distribuer dans ledit réservoir, une tête de pulvérisation amovible étant assemblée sur ledit réservoir, autour de ladite aiguille de prélèvement, pour pulvériser ledit produit fluide à distribuer hors dudit réservoir, ladite tête de pulvérisation comportant un orifice de distribution et un insert disposé en amont dudit orifice de distribution, ladite tête de pulvérisation, lorsqu'assemblée sur ledit réservoir, est déplaçable axialement entre une position de transport et une position d'utilisation, ledit ensemble comportant des moyens de guidage pour guider ladite tête de pulvérisation lors de son assemblage dans sa position de transport et/ou lors de son déplacement dans sa position d'utilisation, des premiers moyens de blocage pour maintenir ladite tête de pulvérisation dans sa position de transport, et des seconds moyens de blocage pour maintenir ladite tête de pulvérisation dans sa position d'utilisation.

Avantageusement, ladite aiguille de prélèvement comporte une partie de canule pourvue d'un orifice de sortie et comportant une première partie de plus grand diamètre et une seconde partie de plus petit diamètre, formant la pointe qui comporte ledit orifice de sortie, ledit insert comportant un manchon de réception recevant ladite pointe lorsque ladite tête de pulvérisation est assemblée sur ledit réservoir.

Avantageusement, dans ladite position de transport, ladite pointe formée par ladite seconde partie de plus petit diamètre est disposée à l'intérieur dudit manchon de réception avec un espace entre la surface externe de ladite pointe et la surface interne dudit manchon de réception.

Avantageusement, dans ladite position d'utilisation, ladite première partie de plus grand diamètre est en contact serrant, notamment étanche, avec ledit manchon de réception.

Avantageusement, dans ladite position d'utilisation, ladite seconde partie de plus petit diamètre est en contact serrant avec ledit manchon de réception.

Selon un mode de réalisation avantageux, ladite tête de pulvérisation comporte au moins un profil interne s'étendant radialement vers l'intérieur et ledit réservoir comporte des seconds et troisièmes profils externes s'étendant radialement vers l'extérieur, décalés axialement sur une surface externe dudit réservoir, ledit au moins un profil interne coopérant avec lesdits seconds profils externes pour former lesdits premiers moyens de blocage définissant ladite position de transport et avec lesdits troisièmes profils externes pour former lesdits seconds moyens de blocage définissant ladite position d'utilisation.

Avantageusement, ladite tête de pulvérisation comporte une pluralité de profils internes, de préférence trois, répartis sur sa périphérie, et lesdits seconds et troisièmes profils externes dudit réservoir sont respectivement formés par une nervure périphérique.

Avantageusement, ledit réservoir comporte des premiers profils externes s'étendant radialement vers l'extérieur, décalés axialement vers le haut par rapport auxdits seconds profils externes, et coopérant avec ladite tête de pulvérisation pour former lesdits moyens de guidage.

En variante, ladite aiguille de prélèvement comporte une paroi radialement externe coopérant avec ladite tête de pulvérisation pour former lesdits moyens de guidage.

Selon un autre mode de réalisation avantageux, ledit réservoir comporte un ergot radialement saillant, ladite tête de pulvérisation comportant une première et une seconde découpes, diamétralement opposées, s'étendant axialement vers le haut à partir d'un bord axialement inférieur, ladite seconde découpe étant plus grande que ladite première découpe, de sorte que lesdits premiers moyens de blocage sont formés par ledit ergot coopérant avec ladite première découpe et lesdits seconds moyens de blocage sont formés par ledit ergot coopérant avec ladite seconde découpe.

Avantageusement, chaque découpe se termine par une zone de clipsage respective dans laquelle ledit ergot vient s'encliqueter.

Avantageusement, ledit réservoir et/ou ledit piston comporte(nt) des moyens de fractionnement de dose pour séparer le produit fluide à distribuer contenu dans ledit réservoir en au moins deux doses destinées à être pulvérisées lors de plusieurs actionnements successifs dudit ensemble.

Avantageusement, lesdits moyens de fractionnement de dose comportent un ergot dudit piston coulissant dans une rainure dudit réservoir, ladite rainure comportant un épaulement pour bloquer ledit ergot après pulvérisation de la première dose de produit fluide.

Avantageusement, ledit réservoir et/ou ledit piston comporte(nt) des moyens d'accumulation d'énergie nécessitant l'application d'au moins une force prédéterminée pour permettre la pulvérisation dudit produit fluide à distribuer.

Avantageusement, lesdits moyens d'accumulation d'énergie comportent au moins un étranglement ou bossage coopérant avec ledit ergot dudit piston, ledit ergot pouvant passer au-delà desdits moyens d'accumulation d'énergie lorsqu'au moins ladite force prédéterminée est appliquée sur ledit piston.

Avantageusement, avant actionnement, ledit ergot est décalé axialement desdits moyens d'accumulation d'énergie, de sorte qu'au début d'une course d'actionnement, ledit piston réalise une purge d'air et/ou de volume mort.

Ces avantages et caractéristiques et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en section transversale d'un ensemble de distribution selon un premier mode de réalisation avantageux, avec la tête de pulvérisation assemblée en position de transport,
La figure 2 est une vue similaire à celle de la figure 1, en position d'utilisation,
La figure 3 est une vue de détail en section transversale de la tête de pulvérisation selon le premier mode de réalisation,
La figure 4 est une vue en section horizontale de la tête de pulvérisation selon la ligne de coupe A-A sur la figure 3
La figure 5 est une vue schématique en perspective de l'insert,
La figure 6 est une vue schématique en perspective de l'aiguille de prélèvement selon le premier mode de réalisation,
La figure 7 est une vue schématique en perspective du réservoir selon le premier mode de réalisation,
La figure 8 est une vue schématique en perspective du piston,
La figure 9 est une vue schématique de l'ensemble en position de transport,
La figure 10 illustre l'ensemble sans la tête de pulvérisation, pour la phase de prélèvement,
Les figures 11 à 13 montrent l'ensemble respectivement avant distribution de la première dose, après distribution de la première dose et avant distribution de la seconde dose et après distribution de la seconde dose,
La figure 14 est une vue schématique en perspective de l'aiguille de prélèvement selon un second mode de réalisation,
La figure 15 est une vue schématique en section transversale d'un ensemble de distribution selon le second mode de réalisation, avec la tête de pulvérisation assemblée en position de transport,
La figure 16 est une vue schématique en perspective du réservoir selon un troisième mode de réalisation, et
La figure 17 est une vue schématique en perspective de la tête de pulvérisation selon le troisième mode de réalisation.

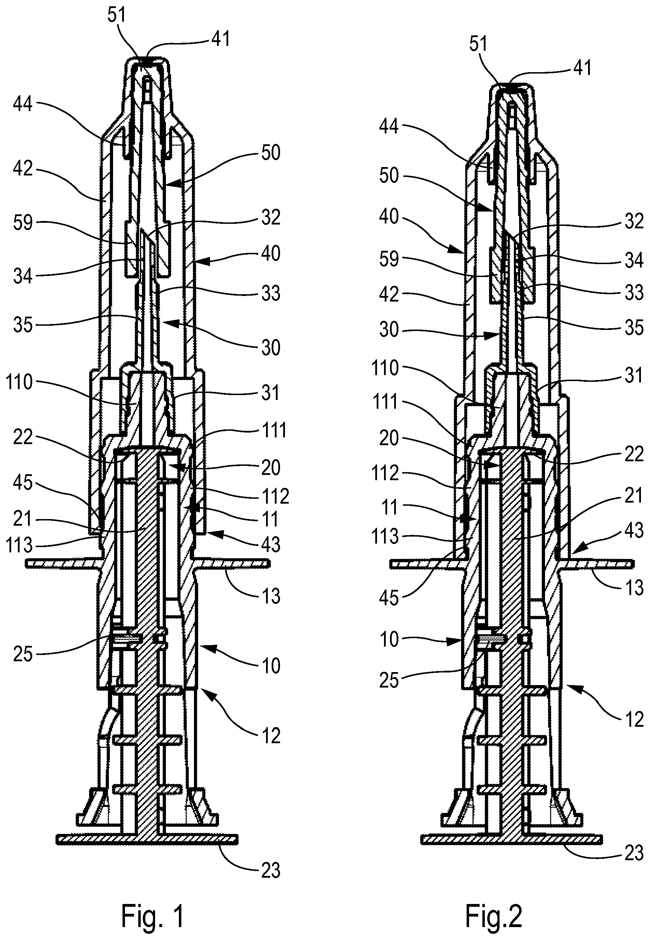

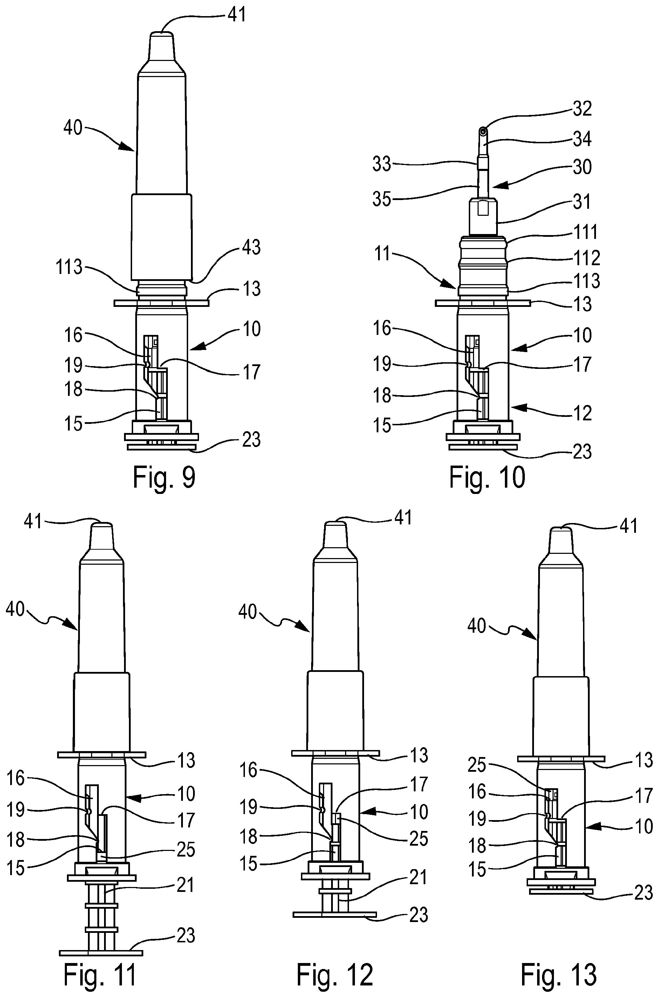

L'ensemble de distribution comporte un réservoir 10 du type seringue et un piston 20 coulissant dans ledit réservoir entre une position rétractée, visible sur la figure 11, et une position enfoncée, qui est celle représentée sur les figures 1, 2, 9, 10, 13 et 15. Une aiguille de prélèvement 30 est fixée sur le réservoir 10, et une tête de pulvérisation 40 pourvue d'un orifice de distribution 41 vient s'assembler de manière amovible sur ladite aiguille de prélèvement 30. La tête de pulvérisation 40 contient un insert creux 50 disposé en amont de l'orifice de distribution 41. En phase de prélèvement, la tête de pulvérisation 40 est enlevée pour exposer l'aiguille de prélèvement 30 qui peut alors être insérée dans un récipient contenant du produit fluide à aspirer dans le réservoir 10 à travers ladite aiguille de prélèvement 30. Après cette phase de prélèvement, la tête de pulvérisation 40 est remise en place, autour de ladite aiguille de prélèvement, ce qui permet de distribuer le produit fluide contenu dans le réservoir 10 sous forme de spray, par exemple pour une pulvérisation nasale.

Selon l'invention, la tête de pulvérisation amovible 40, lorsqu'assemblée sur le réservoir 10, est déplaçable axialement entre une position de transport et une position d'utilisation.

Le piston 20 comporte un élément de piston 22 solidaire d'une tige d'actionnement 21 actionnée par l'utilisateur au moment de la distribution, notamment en appuyant manuellement sur une collerette d'extrémité 23 de la tige de piston 21. L'élément de piston 22 peut être réalisé d'une pièce avec la tige de piston 21, par exemple par moulage d'un matériau plastique, ou former un élément séparé fixé, par exemple surmoulé, à ladite tige de piston. Dans ce cas, l'élément de piston 22 peut être réalisé en un matériau différent de la tige de piston 21.

Le réservoir 10 comporte une partie supérieure 11 destinée à recevoir le produit fluide à distribuer et une partie inférieure 12 destinée à coopérer avec la tige d'actionnement 21 du piston 20. Une bride radiale 13 formant repose-doigts est avantageusement prévue entre lesdites première et seconde parties 11, 12 du réservoir 10.

La partie supérieure 11 du réservoir 10 comporte une partie d'extrémité 110 sur laquelle se fixe l'aiguille de prélèvement 30, par exemple par vissage ou encliquetage. Cette partie d'extrémité 110 peut être de diamètre externe réduit, comme illustré sur les figures 1, 2 et 7.

La partie inférieure 12 du réservoir 10 comporte au moins une rainure 15, qui coopère chacune avec un ergot 25 de ladite tige de piston 21. Cet ergot 25 se projette radialement vers l'extérieur et coulisse dans ladite au moins une rainure 15 lors de l'actionnement dudit ensemble de distribution. Avantageusement, deux rainures diamétralement opposées 15 peuvent être prévues, ainsi que deux ergots 25 diamétralement opposés.

La rainure 15 peut comporter un épaulement 17 formant moyen de fractionnement de dose. Dans ce cas, la rainure 15 se prolonge axialement par une partie supérieure de rainure 16 décalée latéralement. Ainsi, lors de l'actionnement du piston 20 en vue de pulvériser le produit fluide à distribuer, l'ergot 25 du piston 20 coulisse axialement dans une première partie de la rainure 15, et cet ergot 25 est stoppé par ledit épaulement 17 de la rainure 15. Ceci définit la première dose, en coupant la course d'actionnement du piston 20 dans le réservoir 10. L'utilisateur doit alors par exemple tourner légèrement le piston 20 pour amener l'ergot 25 en face de la seconde partie de rainure 16 afin de pouvoir pulvériser la seconde dose. Bien entendu, l'invention s'applique aussi à un dispositif unidose, dans lequel la totalité du produit fluide à distribuer est pulvérisée en une seule dose. Elle peut aussi s'appliquer à des dispositifs contenant plus de deux doses, par exemple trois ou quatre doses.

Avantageusement, la rainure 15 comporte des moyens d'accumulation d'énergie, tels que par exemple des étranglements 18, 19, qui peuvent coopérer avec l'ergot 25 au début de chaque course d'actionnement pour imposer que l'utilisateur exerce au moins une force suffisante sur le piston 20 pour surmonter la résistance générée par lesdits moyens d'accumulation d'énergie. Lorsque cette force est atteinte, l'ergot 25 passe au-delà desdits moyens d'accumulation d'énergie, ce qui libère brusquement l'énergie accumulée dans la main de l'utilisateur, garantissant ainsi que la course d'actionnement, ou la demi-course d'actionnement dans l'exemple représenté d'un bidose, soit réalisée en totalité. Bien entendu, ces moyens d'accumulation d'énergie peuvent être réalisés d'une quelconque manière appropriée, et les étranglements 18, 19 ne sont qu'un exemple de réalisation avantageux. Par exemple on peut envisager des bossages ou des ponts sécables dans ladite rainure 15, 16.

D'autres variantes pour les moyens de fractionnement de dose sont envisageables, par exemple une partie élastiquement déformable formée soit sur le piston, soit sur le réservoir, et qui se déforme lorsqu'une force suffisante est appliquée sur le piston. Dans ce cas, les moyens de fractionnement de dose pourraient former simultanément moyens d'accumulation d'énergie. D'autres variantes sont aussi envisageables.

Dans l'exemple représenté, l'aiguille de prélèvement 30 comporte une partie de canule 35 pourvue d'un orifice de sortie 32. Une partie de fixation 31 est prévue pour fixer l'aiguille de prélèvement 30 sur le réservoir 10, notamment sa partie d'extrémité 110, par exemple par vissage, encliquetage ou tout autre moyen de fixation approprié. La partie de canule 35 comporte avantageusement une première partie 33 de plus grand diamètre reliée à la partie de fixation 31 et une seconde partie 34 de plus petit diamètre, formant la pointe qui comporte l'orifice de sortie 32.

La tête de pulvérisation 40 comporte un corps creux 42 pourvu d'une paroi de fond comportant un orifice de pulvérisation 41.

La tête de pulvérisation 40 contient un insert 50 en amont de l'orifice de pulvérisation 42 pour s'adapter sur l'aiguille du prélèvement 30. Cet insert est avantageusement inséré dans un manchon supérieur 44 de la tête de pulvérisation 40. L'insert 50 comporte avantageusement un ou plusieurs passages latéraux 55 qui permettent au produit fluide de passer de l'intérieur dudit insert 50 vers l'extérieur. La paroi d'extrémité axiale supérieure 51 dudit insert 50 est alors fermée, et la surface externe de ladite paroi d'extrémité axiale 51 dudit insert peut former avec la paroi de fond de la tête de pulvérisation 40, un profil de pulvérisation, comportant par exemple des canaux de tourbillonnement 52 et une chambre de pulvérisation 53. Ce profil de pulvérisation permet de créer une bonne pulvérisation lors de la distribution du produit fluide à travers ladite tête de pulvérisation 40. Le profil de pulvérisation peut être formé sur la surface externe de ladite paroi d'extrémité 51 dudit insert. En variante, le profil de pulvérisation peut être prévu dans la paroi de fond de ladite tête de pulvérisation 40, auquel cas la surface externe de la paroi d'extrémité axiale 51 de l'insert pourrait être lisse. De préférence, ladite tête de pulvérisation 40 et ledit insert 50 sont chacun réalisés par moulage d'un matériau plastique. Avantageusement, l'unité formée par la tête de pulvérisation 40 et l'insert 50 est réalisé tel que décrit dans le document WO2021094683A1.

Du côté opposé à la paroi d'extrémité axiale supérieure 51, l'insert 50 comporte un manchon de réception 59 destiné à recevoir la pointe de l'aiguille de prélèvement 30 lorsque la tête de pulvérisation 40 est assemblée sur le réservoir.

Comme visible sur la figure 1, dans la position de transport, la pointe de l'aiguille formée par la seconde partie 34 de plus petit diamètre de la partie de canule 35 est avantageusement disposée à l'intérieur du manchon de réception 59 de l'insert 50, mais avec un espace entre la surface externe de la pointe 34 et la surface interne du manchon de réception 59. Ainsi, dans cette position de transport, aucune contrainte radiale n'est exercée par l'insert 50 sur la pointe 34, mais celle-ci est quand même protégée par le manchon de réception 59. Ainsi, en cas de chute accidentelle de l'ensemble, par exemple pendant le transport, il n'y a aucun risque d'endommager la pointe 34, même si la tête de pulvérisation 40 subit des contraintes par rapport au réservoir 10 en raison du choc lié à la chute.

Lorsque la tête de pulvérisation 40 est déplacée axialement vers sa position d'utilisation, comme représenté sur la figure 2, la partie de canule 35 pénètre davantage dans le manchon de réception 59, jusqu'à ce que la première partie 33 de l'aiguille vienne avantageusement en contact serrant et étanche avec ledit manchon de réception 59. Avantageusement, le diamètre du manchon de réception 59 diminue en direction de l'orifice de distribution 41, et il est donc possible qu'en position d'utilisation, la pointe formée par la seconde partie 34 de la partie de canule 35 vienne également en contact serrant avec la paroi interne du manchon de réception 59.

Selon l'invention, l'ensemble comporte des moyens de guidage pour guider ladite tête de pulvérisation 40 lors de son assemblage dans sa position de transport et/ou lors de son déplacement dans sa position d'utilisation, des premiers moyens de blocage pour maintenir ladite tête de pulvérisation 40 dans sa position de transport, et des seconds moyens de blocage pour maintenir ladite tête de pulvérisation 40 dans sa position d'utilisation.

Les figures 1 à 13 illustrent un premier mode de réalisation avantageux.

Dans ce premier mode de réalisation, la partie supérieure 11 du réservoir 10 comporte des premiers, seconds et troisièmes profils externes 111, 112, 113 s'étendant radialement vers l'extérieur, décalés axialement sur la surface externe de ladite partie supérieure 11, et destinés à coopérer avec la tête de pulvérisation 40, comme cela sera décrit plus en détails ci-après. Chaque série de profils externes 111, 112, 113 peut comporter un seul profil s'étendant sur la périphérie, par exemple sous la forme d'une nervure. En variante, on pourrait envisager pour chaque série de profils externes une pluralité de projections réparties sur la périphérie.

Le bord axial inférieur 43 de la tête de pulvérisation 40 comporte au moins un profil interne 45, qui s'étend radialement vers l'intérieur. Avantageusement, il y a une pluralité de profils internes 45, répartis sur la circonférence du bord axial inférieur 43, par exemple trois ou quatre. En variante, un seul profil interne s'étendant sur toute la circonférence est possible.

Cet au moins un profil interne 45 coopère lors de l'assemblage de la tête de pulvérisation 40 sur le réservoir 10 avec les premiers, seconds et/ou troisièmes profils externes 111, 112, 113.

Ainsi, lorsque la tête de pulvérisation 40 est assemblée sur le réservoir 10, le au moins un profil interne 45 passe d'abord sur les premiers profils externes 111, qui vont agir principalement de guidage et d'alignement axial de la tête de pulvérisation par rapport au réservoir. Dans ce premier mode de réalisation, les moyens de guidage sont donc formés par les premiers profils externes 111.

Pour le transport et le stockage, le au moins un profil interne 45 passe sur les seconds profils externes 112, mais pas sur les troisièmes profils externes 113, comme visible sur la figure 1. Dans ce premier mode de réalisation, les premiers moyens de blocage sont donc formés par les seconds profils externes 112.

Ce n'est que lorsque le dispositif doit être utilisé pour pulvériser le contenu du réservoir que le au moins un profil interne 45 passe sur les troisièmes profils externes 113 pour s'y encliqueter. Dans ce premier mode de réalisation, les seconds moyens de blocage sont donc formés par les troisièmes profils externes 113.

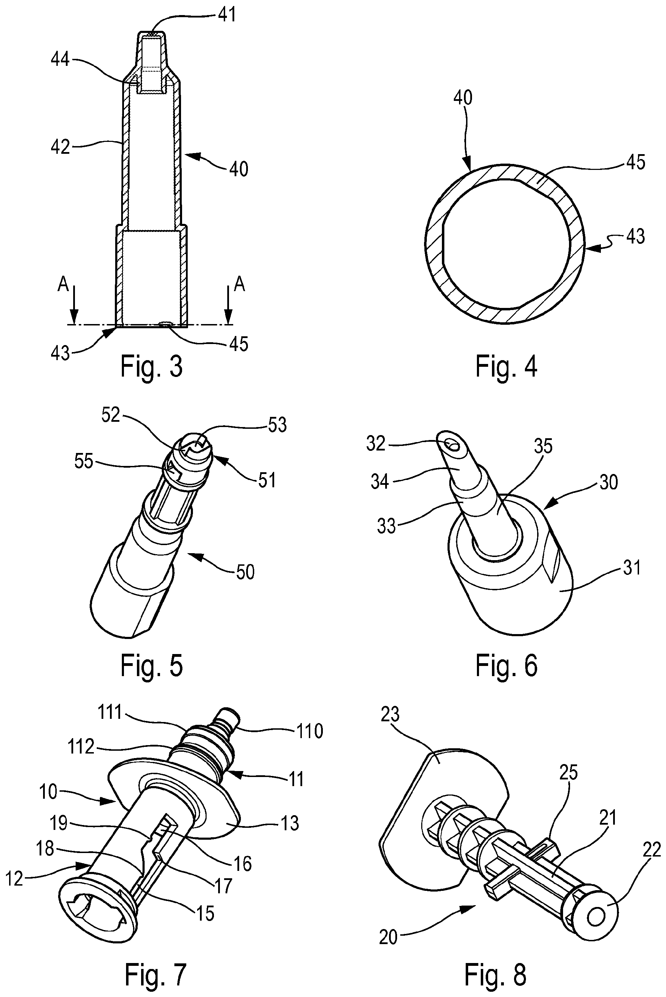

Dans l'exemple représenté sur les figures 1 à 13, il y a trois profils internes 45 répartis sur la périphérie de la tête de pulvérisation 40, comme visible sur la figure 4. Dans ce cas, les premiers, seconds et troisièmes profils externes 111, 112, 113 du réservoir sont respectivement formés par une nervure périphérique, comme visible sur la figure 10.

Avantageusement, chaque pièce formant l'ensemble de distribution peut être réalisé en un matériau spécifique adapté pour optimiser ses propriétés.

Ainsi, par exemple, la tête de pulvérisation 40 peut être réalisée en un matériau rigide mais présentant néanmoins une légère flexibilité, tel que du polypropylène, pour lui permettre de s'encliqueter sur les profils externes 111, 112, 113 du réservoir 10.

De même, l'insert 50 peut être réalisé en un matériau rigide mais présentant néanmoins une légère flexibilité, tel que du polypropylène, pour lui permettre de réaliser l'étanchéité d'une part avec la tête de pulvérisation 40 et d'autre part avec l'aiguille de prélèvement 30.

Le réservoir 10 peut être réalisée en un matériau rigide et suffisamment translucide pour permettre de voir le produit fluide dans réservoir, mais présentant néanmoins une légère flexibilité, tel que du polypropylène, pour permettre un encliquetage dans la tête de pulvérisation 40 avec un effort limité en position d'utilisation.

Avantageusement, la tête de pulvérisation 40, l'insert 50 et le réservoir 10 sont réalisés dans le même matériau, de préférence du polypropylène.

L'aiguille de prélèvement 30 est avantageusement réalisée en un matériau suffisamment fluide au moulage, mais rigide et non cassant après refroidissement pour permettre de percer efficacement, par exemple du COC, COP, PTCA, PBT ou PC.

Le piston 20 peut être réalisée en un matériau rigide et avec un état de surface lisse et glissant, mais présentant néanmoins une légère flexibilité, tel que du polyéthylène haute densité, pour permettre d'assurer l'étanchéité avec le réservoir 10 sans générer trop d'effort. Le compromis rigidité/flexibilité est également important pour l'assemblage du piston 20 dans le réservoir 10 et pour une accumulation d'énergie efficace à chaque actionnement.

Les figures 9 à 13 illustrent partiellement les phases d'utilisation d'un ensemble de distribution selon le premier mode de réalisation tel que décrit ci-dessus.

Comme visible sur la figure 9, avant utilisation, la tête de pulvérisation 40 est préassemblée de manière amovible dans sa position de stockage et de transport sur ladite tête de prélèvement 30, dans laquelle la pointe de l'aiguille ne subit pas de contraintes tout en étant protégé dans le manchon de réception 59 de l'insert 50.

L'utilisateur peut alors retirer ladite tête de pulvérisation 40, comme illustré sur la figure 10, pour réaliser la phase de prélèvement, puis la remettre ensuite en position d'utilisation, pour réaliser la ou les pulvérisations. Le prélèvement est réalisé de manière classique en faisant coulisser le piston 20 dans le réservoir 10 en éloignement de ladite aiguille 35, aspirant ainsi le fluide depuis ledit réservoir de prélèvement dans ledit réservoir 10. Eventuellement, plusieurs fluides différents peuvent être aspirés à partir de plusieurs réservoirs de prélèvement différents, pour se mélanger dans le réservoir 10, avant pulvérisation.

La tête de pulvérisation 40 est alors à nouveau assemblée autour de ladite aiguille de prélèvement 30, en position d'utilisation, comme visible sur la figure 11.

Le produit fluide à distribuer peut alors être pulvérisé à travers ladite tête de pulvérisation 40.

Avantageusement, avant la réalisation de la première et/ou de la seconde dose, une purge, par exemple d'air et d'un volume mort, peut être réalisée avant la distribution. Ainsi, comme visible sur la figure 11, au repos, l'ergot 25 de la tige de piston est décalé axialement de l'étranglement 18. Au début de la course d'actionnement, jusqu'à ce que l'ergot 25 de la tige de piston entre en contact avec l'étranglement 18 permettant l'accumulation d'énergie, le déplacement axial du piston permet de réaliser cette purge.

La figure 12 montre l'ensemble après distribution de la première dose et la figure 13 montre l'ensemble après distribution de la seconde dose.

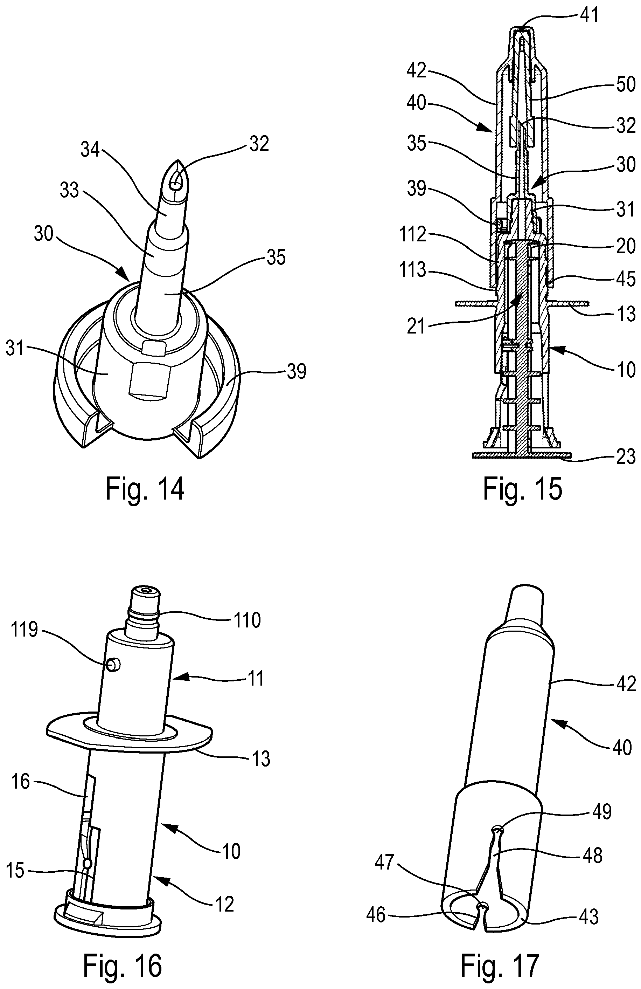

Les figures 14 et 15 illustrent un second mode de réalisation, qui ne diffère du premier mode de réalisation décrit ci-dessus que par les moyens de guidage.

Dans ce second mode de réalisation, il n'y plus les premiers profils externes 111 sur le réservoir, et c'est l'aiguille de prélèvement 30 qui comporte une paroi radialement externe 39 qui coopère avec l'intérieur de la tête de pulvérisation 40 pour réaliser son guidage lors de son déplacement axial.

Les figures 16 et 17 illustrent un troisième mode de réalisation.

Dans ce troisième mode de réalisation, les moyens de guidage sont comme dans le second mode de réalisation formés par la paroi 39 de l'aiguille de prélèvement 30.

La différence ici concerne les premiers et seconds moyens de blocage.

Ainsi, le réservoir 10 comporte dans sa partie supérieure 11 un ergot 119 radialement saillant.

La tête de pulvérisation 40 comporte deux découpes 46, 48, diamétralement opposées s'étendant axialement vers le haut à partir du bord axialement inférieur 43. La seconde découpe 48 est plus grande que la première découpe 46. Avantageusement, chaque découpe 46, 48 se termine par une zone de clipsage 47, 49 dans laquelle l'ergot 119 du réservoir peut venir s'encliqueter.

Ainsi, en position de transport, l'ergot 119 vient se clipser dans la zone de clipsage 47 de la première découpe 46.

Lorsque l'utilisateur souhaite utiliser l'ensemble, notamment en phase de prélèvement, il va retirer la tête de pulvérisation 40 de sa position de transport.

Ensuite, avant utilisation de l'ensemble pour distribuer la ou les doses de produit fluide contenu dans le réservoir 10 après la phase de prélèvement, la tête de pulvérisation 40 va être mise en place, mais cette fois avec l'ergot 119 du réservoir qui pénètre dans la seconde découpe 48 pour venir se clipser en position d'utilisation dans la zone de clipsage 49.

D'autres variantes de réalisation pour les moyens de guidage, les premiers moyens de blocage et les seconds moyens de blocage sont envisageables.

Bien que la présente invention ait été décrite en référence à plusieurs modes de réalisation avantageux de celle-ci, il est entendu qu'un homme du métier peut y apporter toutes modifications utiles, sans sortir du cadre de la présente invention tel que défini par les revendications annexées.

## Revendications

1. Ensemble de distribution de produit fluide comportant un réservoir (10) destiné à contenir un produit fluide et un piston (20) coulissant dans ledit réservoir (10), une aiguille de prélèvement (30) étant fixée sur ledit réservoir (10) pour aspirer un produit fluide à distribuer dans ledit réservoir (10), une tête de pulvérisation (40) amovible étant assemblée sur ledit réservoir (10), autour de ladite aiguille de prélèvement (30), pour pulvériser ledit produit fluide à distribuer hors dudit réservoir (10), ladite tête de pulvérisation (40) comportant un orifice de distribution (41) et un insert (50) disposé en amont dudit orifice de distribution (41), ladite tête de pulvérisation (40), lorsqu'assemblée sur ledit réservoir (10), étant déplaçable axialement entre une position de transport et une position d'utilisation, ledit ensemble comportant des moyens de guidage (111; 39) pour guider ladite tête de pulvérisation (40) lors de son assemblage dans sa position de transport et/ou lors de son déplacement dans sa position d'utilisation, des premiers moyens de blocage (112; 46, 119) pour maintenir ladite tête de pulvérisation (40) dans sa position de transport,
ledit ensemble étant **caractérisé en ce qu'**il comporte des seconds moyens de blocage (113; 48, 119) pour maintenir ladite tête de pulvérisation (40) dans sa position d'utilisation.

2. Ensemble selon la revendication 1, dans lequel ladite aiguille de prélèvement (30) comporte une partie de canule (35) pourvue d'un orifice de sortie (32) et comportant une première partie (33) de plus grand diamètre et une seconde partie (34) de plus petit diamètre, formant la pointe qui comporte ledit orifice de sortie (32), ledit insert (50) comportant un manchon de réception (59) recevant ladite pointe lorsque ladite tête de pulvérisation (40) est assemblée sur ledit réservoir (10).

3. Ensemble selon la revendication 2, dans lequel, dans ladite position de transport, ladite pointe formée par ladite seconde partie (34) de plus petit diamètre est disposée à l'intérieur dudit manchon de réception (59) avec un espace entre la surface externe de ladite pointe et la surface interne dudit manchon de réception.

4. Ensemble selon la revendication 2 ou 3, dans lequel, dans ladite position d'utilisation, ladite première partie (33) de plus grand diamètre est en contact serrant, notamment étanche, avec ledit manchon de réception (59).

5. Ensemble selon l'une quelconque des revendications 2 à 4, dans lequel, dans ladite position d'utilisation, ladite seconde partie (34) de plus petit diamètre est en contact serrant avec ledit manchon de réception (59).

6. Ensemble selon l'une quelconque des revendications précédentes, dans lequel ladite tête de pulvérisation (40) comporte au moins un profil interne (45) s'étendant radialement vers l'intérieur et ledit réservoir (10) comporte des seconds et troisièmes profils externes (112, 113) s'étendant radialement vers l'extérieur, décalés axialement sur une surface externe dudit réservoir (10), ledit au moins un profil interne (45) coopérant avec lesdits seconds profils externes (112) pour former lesdits premiers moyens de blocage définissant ladite position de transport et avec lesdits troisièmes profils externes (113) pour former lesdits seconds moyens de blocage définissant ladite position d'utilisation.

7. Ensemble selon la revendication 6, dans lequel ladite tête de pulvérisation (40) comporte une pluralité de profils internes (45), de préférence trois, répartis sur sa périphérie, et lesdits seconds et troisièmes profils externes (112, 113) dudit réservoir (10) sont respectivement formés par une nervure périphérique.

8. Ensemble selon la revendication 6 ou 7, dans lequel ledit réservoir (10) comporte des premiers profils externes (111) s'étendant radialement vers l'extérieur, décalés axialement vers le haut par rapport auxdits seconds profils externes (112), et coopérant avec ladite tête de pulvérisation (40) pour former lesdits moyens de guidage.

9. Ensemble selon la revendication 6 ou 7, dans lequel ladite aiguille de prélèvement (30) comporte une paroi radialement externe (39) coopérant avec ladite tête de pulvérisation (40) pour former lesdits moyens de guidage.

10. Ensemble selon l'une quelconque des revendications 1 à 5, dans lequel ledit réservoir (10) comporte un ergot (119) radialement saillant, ladite tête de pulvérisation (40) comportant une première et une seconde découpes (46, 48), diamétralement opposées, s'étendant axialement vers le haut à partir d'un bord axialement inférieur (43), ladite seconde découpe (48) étant plus grande que ladite première découpe (46), de sorte que lesdits premiers moyens de blocage sont formés par ledit ergot (119) coopérant avec ladite première découpe (46) et lesdits seconds moyens de blocage sont formés par ledit ergot (119) coopérant avec ladite seconde découpe (48).

11. Ensemble selon la revendication 10, dans lequel chaque découpe (46, 48) se termine par une zone de clipsage respective (47, 49) dans laquelle ledit ergot (119) vient s'encliqueter.

12. Ensemble selon l'une quelconque des revendications précédentes, dans lequel ledit réservoir (10) et/ou ledit piston (20) comporte(nt) des moyens de fractionnement de dose (15, 17 ; 25) pour séparer le produit fluide à distribuer contenu dans ledit réservoir (10) en au moins deux doses destinées à être pulvérisées lors de plusieurs actionnements successifs dudit ensemble.

13. Ensemble selon la revendication 12, dans lequel lesdits moyens de fractionnement de dose comportent un ergot (25) dudit piston (20) coulissant dans une rainure (15) dudit réservoir (10), ladite rainure comportant un épaulement (17) pour bloquer ledit ergot (25) après pulvérisation de la première dose de produit fluide.

14. Ensemble selon l'une quelconque des revendications précédentes, dans lequel ledit réservoir (10) et/ou ledit piston (20) comporte(nt) des moyens d'accumulation d'énergie (18, 19) nécessitant l'application d'au moins une force prédéterminée pour permettre la pulvérisation dudit produit fluide à distribuer.

15. Ensemble selon les revendications 13 et 14, dans lequel lesdits moyens d'accumulation d'énergie (18, 19) comportent au moins un étranglement ou bossage coopérant avec ledit ergot (25) dudit piston, ledit ergot (25) pouvant passer au-delà desdits moyens d'accumulation d'énergie lorsqu'au moins ladite force prédéterminée est appliquée sur ledit piston (20).

16. Ensemble selon la revendication 15, dans lequel, avant actionnement, ledit ergot (25) est décalé axialement desdits moyens d'accumulation d'énergie (18, 19), de sorte qu'au début d'une course d'actionnement, ledit piston (20) réalise une purge d'air et/ou de volume mort.

## Patentansprüche

1. Anordnung zur Abgabe eines fluiden Produkts, aufweisend einen Behälter (10), der bestimmt ist, ein fluides Produkt zu enthalten, und einen in diesem Behälter (10) gleitenden Kolben (20), wobei an dem Behälter (10) eine Entnahmenadel (30) zum Ansaugen eines abzugebenden fluiden Produkts in dem Behälter (10) befestigt ist, wobei ein abnehmbaren Sprühkopf (40) an dem Behälter (10) um die Entnahmenadel (30) herum angebracht ist, um das abzugebende fluide Produkt aus dem Behälter (10) zu sprühen, wobei der Sprühkopf (40) eine Abgabeöffnung (41) und einen Einsatz (50) aufweist, der stromaufwärts der Abgabeöffnung (41) angeordnet ist, wobei der Sprühkopf (40), wenn er an dem Behälter (10) angebracht ist, zwischen einer Transportstellung und einer Gebrauchsstellung axial verlagerbar ist, wobei die Anordnung Führungsmittel (111; 39) zum Führen des Sprühkopfes (40) bei seiner Anbringung in seiner Transportstellung und/oder bei seiner Verlagerung in seine Gebrauchsstellung, erste Verriegelungsmittel (112; 46, 119) zum Halten des Sprühkopfes (40) in seiner Transportstellung aufweist, wobei die Anordnung **dadurch gekennzeichnet ist, dass** sie zweite Verriegelungsmittel (113; 48, 119) zum Halten des Sprühkopfes (40) in seiner Gebrauchsstellung aufweist.

2. Anordnung nach Anspruch 1, wobei die Entnahmenadel (30) einen Kanülenabschnitt (35) mit einer Austrittsöffnung (32) aufweist und einen ersten Abschnitt (33) mit größerem Durchmesser und einen zweiten Abschnitt (34) mit kleinerem Durchmesser aufweist, der die Spitze bilden, die die Austrittsöffnung (32) aufweist, wobei der Einsatz (50) eine Aufnahmehülse (59) aufweist, die die Spitze aufnimmt, wenn der Sprühkopf (40) auf dem Behälter angebracht ist.

3. Anordnung nach Anspruch 2, wobei in der Transportstellung die durch den zweiten Abschnitt (34) mit kleinerem Durchmesser gebildete Spitze in der Aufnahmehülse (59) mit einem Zwischenraum zwischen der Außenfläche der Spitze und der Innenfläche der Aufnahmehülse angeordnet ist.

4. Anordnung nach Anspruch 2 oder 3, wobei in der Gebrauchsstellung der erste Abschnitt (33) mit größerem Durchmesser in engem, insbesondere dichtem Kontakt mit der Aufnahmehülse (59) steht.

5. Anordnung nach einem der Ansprüche 2 bis 4, wobei in der Gebrauchsstellung der zweite Abschnitt (34) mit kleinerem Durchmesser in engem Kontakt mit der Aufnahmehülse (59) steht.

6. Anordnung nach einem der vorstehenden Ansprüche, wobei der Sprühkopf (40) mindestens ein sich radial nach innen erstreckendes Innenprofil (45) aufweist und der Behälter (10) zweite und dritte Außenprofile (112, 113) aufweist, die sich radial nach außen erstrecken und auf einer Außenfläche des Behälters (10) axial versetzt sind, wobei das mindestens eine Innenprofil (45) mit den zweiten Außenprofilen (112) zusammenwirkt, um die ersten Verriegelungsmittel zu bilden, die die Transportstellung definieren, und mit den dritten Außenprofilen (113), um die zweiten Verriegelungsmittel zu bilden, die die Gebrauchsstellung definieren.

7. Anordnung nach Anspruch 6, wobei der Sprühkopf (40) eine Vielzahl, vorzugsweise drei, auf seinem Umfang verteilte Innenprofile (45) aufweist und die zweiten und dritten Außenprofile (112, 113) des Behälters (10) jeweils durch eine Umfangsrippe gebildet sind.

8. Anordnung nach Anspruch 6 oder 7, wobei der Behälter (10) erste Außenprofile (111) aufweist, die sich radial nach außen erstrecken, axial nach oben in Bezug auf die zweiten Außenprofile (112) versetzt sind und mit dem Sprühkopf (40) zusammenwirken, um die Führungsmittel zu bilden.

9. Anordnung nach Anspruch 6 oder 7, wobei die Entnahmenadel (30) eine radiale Außenwand (39) aufweist, die mit dem Sprühkopf (40) zusammenwirkt, um die Führungsmittel zu bilden.

10. Anordnung nach einem der Ansprüche 1 bis 5, wobei der Behälter (10) einen radial vorstehenden Stift (119) aufweist, wobei der Sprühkopf (40) einen ersten und einen zweiten Ausschnitt (46, 48) aufweist, die diametral gegenüberliegen, die sich axial nach oben von einer axial unteren Kante (43) aus erstrecken, wobei der zweite Ausschnitt (48) größer ist als der erste Ausschnitt (46), so dass die ersten Verriegelungsmittel durch den Stift (119) gebildet werden, der mit dem ersten Ausschnitt (46) zusammenwirkt und die zweiten Verriegelungsmittel durch den Stift (119) gebildet werden, der mit dem zweiten Ausschnitt (48) zusammenwirkt.

11. Anordnung nach Anspruch 10, wobei jeder Ausschnitt (46, 48) in einem jeweiligen Einrastbereich (47, 49) endet, in den der Stift (119) einrastet.

12. Anordnung nach einem der vorstehenden Ansprüche, wobei der Behälter (10) und/oder der Kolben (20) Dosisteilmittel (15, 17; 25) zum Trennen des in dem Behälter (10) enthaltenen abzugebenden fluiden Produkts (19) in mindestens zwei Dosen aufweist/aufweisen, die bei mehreren aufeinanderfolgenden Betätigungen der Anordnung versprüht werden sollen.

13. Anordnung nach Anspruch 12, wobei die Dosisteilmittel einen Stift (25) des Kolbens (20) aufweisen, der in einer Nut (15) des Behälters (10) gleitet, wobei die Nut einen Absatz (17) aufweist, um den Stift (25) nach dem Versprühen der ersten Dosis des fluiden Produkts zu blockieren.

14. Anordnung nach einem der vorstehenden Ansprüche, wobei der Behälter (10) und/oder der Kolben (20) Energiespeichermittel (18, 19) aufweist/aufweisen, die die Anwendung mindestens einer vorbestimmten Kraft erfordern, um das Versprühen des abzugebenden fluiden Produkts zu ermöglichen.

15. Anordnung nach den Ansprüchen 13 und 14, wobei die Energiespeichermittel (18, 19) mindestens eine Verengung oder Ausbuchtung aufweisen, die mit dem Stift (25) des Kolbens zusammenwirkt, wobei der Stift (25) an den Energiespeichermitteln vorbeilaufen kann, wenn mindestens die vorbestimmte Kraft auf den Kolben (20) ausgeübt wird.

16. Vorrichtung nach Anspruch 15, wobei vor der Betätigung der Stift (25) axial zu den Energiespeichermitteln (18, 19) versetzt wird, so dass zu Beginn eines Betätigungswegs der Kolben (20) eine Entlüftung und/oder einen Totraumausgleich durchführt.

## Claims

1. Assembly for dispensing a fluid product comprising a tank (10) intended to contain a fluid product and a piston (20) sliding in said tank (10), a sampling needle (30) being attached to said tank (10) for drawing a fluid product to be dispensed in said tank (10), a removable spray head (40) being assembled on said tank (10), around said sampling needle (30), to spray said fluid product to be dispensed outside of said tank (10), said spray head (40) comprising a dispensing port (41) and an insert (50) arranged upstream of said dispensing port (41), said spray head (40), when assembled on said tank (10), being axially movable between a transport position and a use position, said assembly comprising guide means (111; 39) for guiding said spray head (40) when it is assembled in its transport position and/or when it is moved into its use position, first locking means (112; 46, 119) for holding said spray head (40) in its transport position, said assembly being **characterized in that** it comprises second locking means (113; 48, 119) for holding said spray head (40) in its use position.

2. Assembly according to claim 1, wherein said sampling needle (30) comprises a cannula part (35) provided with an outlet port (32) and comprising a first part (33) of a larger diameter and a second part (34) of a smaller diameter, forming the tip which comprises said outlet port (32), said insert (50) comprising a receiving sleeve (59) receiving said tip when said spray head (40) is assembled on said tank (10).

3. Assembly according to claim 2, wherein, in said transport position, said tip formed by said second part (34) of a smaller diameter is arranged inside said receiving sleeve (59) with a space between the external surface of said tip and the internal surface of said receiving sleeve.

4. Assembly according to claim 2 or 3, wherein, in said use position, said first part (33) of a larger diameter is in close, in particular sealed contact, with said receiving sleeve (59).

5. Assembly according to any one of claims 2 to 4, wherein, in said use position, said second part (34) of a smaller diameter is in close contact with said receiving sleeve (59).

6. Assembly according to any one of the preceding claims, wherein said spray head (40) comprises at least one internal profile (45) extending radially inwards and said tank (10) comprises second and third external profiles (112, 113) extending radially outwards, axially offset over an external surface of said tank (10), said at least one internal profile (45) engaging with said second external profiles (112) to form said first locking means defining said transport position and with said third external profiles (113) to form said second locking means defining said use position.

7. Assembly according to claim 6, wherein said spray head (40) comprises a plurality of internal profiles (45), preferably three, distributed over its periphery, and said second and third external profiles (112, 113) of said tank (10) are respectively formed by a peripheral ridge.

8. Assembly according to claim 6 or 7, wherein said tank (10) comprises first external profiles (111) extending radially outwards, axially offset upwards with respect to said second external profiles (112), and engaging with said spray head (40) to form said guide means.

9. Assembly according to claim 6 or 7, wherein said sampling needle (30) comprises a radially external wall (39) engaging with said spray head (40) to form said guide means.

10. Assembly according to any one of claims 1 to 5, wherein said tank (10) comprises a radially projecting lug (119), said spray head (40) comprising a first and a second cutout (46, 48), diametrically opposite, extending axially upwards from an axially lower edge (43), said second cutout (48) being larger than said first cutout (46), such that said first locking means are formed by said lug (119) engaging with said first cutout (46) and said second locking means are formed by said lug (119) engaging with said second cutout (48).

11. Assembly according to claim 10, where each cutout (46, 48) ends by a respective clipping zone (47, 49), wherein said lug (119) is snap-fitted.

12. Assembly according to any one of the preceding claims, wherein said tank (10) and/or said piston (20) comprise(s) dose-fractioning means (15, 17; 25) to separate the fluid product to be dispensed contained in said tank (10) into at least two doses intended to be sprayed during several successive actuations of said assembly.

13. Assembly according to claim 12, wherein said dose-fractioning means comprise a lug (25) of said piston (20) sliding in a groove (15) of said tank (10), said groove comprising a shoulder (17) for blocking said lug (25) after spraying of the first dose of fluid product.

14. Assembly according to any one of the preceding claims, wherein said tank (10) and/or said piston (20) comprise(s) energy accumulation means (18, 19) requiring the application of at least one predetermined force to enable the spraying of said fluid product to be dispensed.

15. Assembly according to claims 13 and 14, wherein said energy accumulation means (18, 19) comprise at least one bottleneck or boss engaging with said lug (25) of said piston, said lug (25) being able to pass beyond said energy accumulation means, when at least said predetermined force is applied on said piston (20).

16. Assembly according to claim 15, wherein, before actuation, said lug (25) is axially offset from said energy accumulation means (18, 19), such that at the start of an actuation stroke, said piston (20) performs an air and/or dead volume purge.
